(19) Europäisches Patentamt / European Patent Office / Office européen des brevets

(11) **EP 2 590 622 B1**

(12) **FASCICULE DE BREVET EUROPEEN**

(45) Date de publication et mention
de la délivrance du brevet:
**12.08.2015 Bulletin 2015/33**

(21) Numéro de dépôt: **11728618.7**

(22) Date de dépôt: **24.06.2011**

(51) Int Cl.:
*A61K 36/76* (2006.01)          *A61Q 19/02* (2006.01)
*A61K 8/97* (2006.01)           *A61Q 5/08* (2006.01)

(86) Numéro de dépôt international:
**PCT/EP2011/060603**

(87) Numéro de publication internationale:
**WO 2012/004139 (12.01.2012 Gazette 2012/02)**

(54) **UTILISATION D'UN EXTRAIT DE POPULUS BALSAMIFERA COMME AGENT DÉPIGMENTANT**

VERWENDUNG VON POPULUS BALSAMIFERA-EXTRAKTEN ALS DEPIGMENTIERMITTEL

USE OF POPULUS BALSAMIFERA EXTRACT AS DEPIGMENTING AGENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.07.2010 FR 1055442**

(43) Date de publication de la demande:
**15.05.2013 Bulletin 2013/20**

(73) Titulaire: **Gattefosse S.A.S.
69800 Saint Priest (FR)**

(72) Inventeurs:
- **CHARTON, Virginie
  F-69100 Villeurbanne (FR)**
- **PORTES, Pascal
  F-69008 Lyon (FR)**
- **DEMARNE, Frédéric
  F-13008 Marseille (FR)**

(74) Mandataire: **Denjean, Eric et al
Cabinet Laurent & Charras
"Le Contemporain"
50, Chemin de la Bruyère
69574 Dardilly Cedex (FR)**

(56) Documents cités:
**FR-A1- 2 793 141     US-A1- 2007 071 698**

- **DATABASE WPI Week 200415 Thomson
  Scientific, London, GB; AN 2004-152841
  XP002624774, & KR 2003 079 111 A (KYONGBUK
  COLLEGE SCI) 10 octobre 2003 (2003-10-10) cité
  dans la demande**
- **PEARL I A ET AL: "Studies on the leaves of the
  family salicaceae-xi", PHYTOCHEMISTRY,
  PERGAMON PRESS, GB, vol. 7, no. 10, 1 octobre
  1968 (1968-10-01), pages 1845-1849,
  XP026730369, ISSN: 0031-9422 [extrait le
  1968-10-01] cité dans la demande**
- **YEON HEE KONG, YOUN OCK JO, CHANG-WON
  CHO, DONGWOOK SON, SOOJIN PARK,
  JEONGHAE RHO AND SANG YOON CHOI:
  "Inhibitory Effects of Cinnamic Acid on Melanin
  Biosynthesis in Skin", BIOLOGICAL &
  PHARMACEUTICAL BULLETIN, vol. 31, no. 5, 21
  février 2008 (2008-02-21), pages 946-948,
  XP002624485, cité dans la demande**
- **DATABASE GNPD [Online] Mintel; avril 2010
  (2010-04), "Concentrated Eye Cream",
  XP007917336, Database accession no. 1320826**

**EP 2 590 622 B1**

**Description**

L'invention concerne l'utilisation d'un extrait éthanolique, hydro-éthanolique ou dioxyde de carbone supercritique de *Populus balsamifera,* plus particulièrement d'un extrait de bourgeons, comme agent dépigmentant et/ou anti-pigmentant de la peau et/ou des cheveux et/ou des poils dans une composition cosmétique et/ou dermatologique appliquée par voie topique. L'invention a également pour objet un procédé de traitement cosmétique et/ou dermatologique de dépigmentation et/ou anti-pigmentation de la peau et/ou des cheveux et/ou des poils consistant à appliquer par voie topique la composition contenant ledit extrait de *Populus balsamifera.*

Dans la suite de la description et dans les revendications, par l'expression agent « dépigmentant et/ou anti-pigmentant », on désigne un agent apte à dépigmenter et/ou blanchir la peau (pigmentation constitutive), à éliminer les taches pigmentaires et de sénescence, mais également un agent anti-pigmentant, c'est à dire apte à ralentir le brunissement cutané (pigmentation UV induite), ou à freiner voire à éliminer l'apparition des lentigos solaires. De plus on désigne également cet agent dépigmentant comme apte à dépigmenter et/ou blanchir les cheveux et les poils.

La couleur de la peau a deux origines : la pigmentation et la vascularisation. La pigmentation est due à la synthèse de pigments naturels, les mélanines, par des cellules hautement spécialisées présentes au niveau de la couche basale de l'épiderme, les mélanocytes. Les mélanines assurent la protection de la peau vis à vis des effets néfastes des rayons UV tout en lui conférant sa couleur. Il faut également noter que les mélanines sont également responsables de la couleur des poils et des cheveux.

Les mélanines sont synthétisées au cours d'un processus biochimique complexe, la mélanogenèse, dans des organites spécifiques, les mélanosomes. On distingue 2 types de mélanines, les phaeomélanines de couleur claire/jaune/orangé, et les eumélanines de couleur brune/noire. Selon le phototype d'une personne, les proportions entre phéomélanines et eumélanines varient. Lors de la mélanogenèse, les mélanines s'accumulent dans les mélanosomes pour ensuite migrer via les dendrites des mélanocytes vers les kératinocytes voisins, expliquant ainsi la couleur uniforme de la peau.

Les deux types de mélanines nécessitent pour leur synthèse, un précurseur commun, la tyrosine. Une enzyme, la tyrosinase, transforme la tyrosine en DOPA (3'4-dihydrophénylalanine), puis en DOPAquinone. A partir de la DOPAquinone, deux voies sont possibles : avec l'incorporation de cystéine, la DOPAquinone est transformée en cystéinyl-DOPA, intermédiaire de la synthèse de phaeomélanine ; sans incorporation de cystéine, c'est l'indole 5-6 quinone qui est formé, aboutissant ensuite à l'eumélanine.

La pigmentation cutanée est gouvernée par 150 gènes environ, impliquant les 3 grands types cellulaires de la peau, mélanocytes, kératinocytes et fibroblastes. La pigmentation est principalement régulée par les rayons UV, qui la stimulent indirectement via le relargage kératinocytaire d'$\alpha$-MSH ($\alpha$-Melanocyte Stimulating Hormone).

Au cours du vieillissement, apparaissent sur le dos des mains, le visage, les avant bras, des taches pigmentaires plus foncées et/ou plus colorées (lentigos séniles ou taches de vieillesse), conférant à la peau son hétérogénéité pigmentaire caractéristique du sujet âgé. On distingue également les lentigos solaires ou actiniques, qui ne sont pas liés à l'âge mais à l'exposition solaire répétée, qu'il y ait coup de soleil ou non. Ces lentigos peuvent en effet apparaître dès l'adolescence sur des zones très exposées comme la nuque ou les épaules. Globalement, ces tâches sont dues à une concentration localisée de mélanine à la surface cutanée, liée à l'hyperactivité des mélanocytes ou à leur prolifération bénigne.

L'hyperpigmentation de la grossesse ou chloasma (« masque de grossesse ») est également liée à une hyperactivité des mélanocytes due à l'intense production d'hormones sexuelles durant cette période. Enfin, certaines hyperpigmentations peuvent être dues à une photosensibilisation ou à une cicatrisation post lésionnelle. On comprend donc bien le besoin de substances dépigmentantes topiques, inoffensives et efficaces, en vue de traiter ces hyperpigmentations localisées. De même, dans le cas de certaines leucodermies comme le vitiligo, à défaut de pouvoir repigmenter les peaux lésées, on finit de dépigmenter les zones de peau normale adjacentes pour donner à l'ensemble de la peau une teinte blanche plus homogène. Il ne s'agit donc pas ici d'un traitement thérapeutique visant à traiter le vitiligo, mais d'un traitement cosmétique visant à dépigmenter les zones saines normalement pigmentées voisines des zones pathologiques, en vue d'améliorer l'esthétique général de la peau.

Par ailleurs, certaines personnes qui ont des poils ou des cheveux foncés souhaitent voir ces derniers plus clairs. Ceci peut être particulièrement intéressant pour des raisons esthétiques puisque les poils plus clairs sont moins visibles que lorsqu'ils sont foncés. De même, certaines personnes souhaitent avoir une chevelure avec une teinte blanche plus homogène, lorsque celle-ci commence à blanchir partiellement avec le vieillissement.

Une substance est définie comme dépigmentante et/ou anti-pigmentante lorsqu'elle induit une diminution du taux de mélanine. Plus précisément un agent dépigmentant va entrainer la diminution du taux basal de mélanine alors qu'un agent anti-pigmentant va prévenir la néosynthèse de mélanine induite par les UV. Cette substance dépigmentante et/ou anti-pigmentante peut agir directement sur la viabilité des mélanocytes, ou perturber la mélanogenèse, soit en inhibant une ou plusieurs enzymes impliquées dans la cascade de synthèse, soit en s'intercalant comme analogue structural d'un composé de la chaîne de synthèse des pigments mélaniques.

Parmi les substances les plus utilisées, on trouve l'hydroquinone et ses dérivés qui présentent une bonne efficacité mais par toxicité vis à vis des mélanocytes. L'hydroquinone peut en outre entraîner des dépigmentations permanentes et/ou inégales d'où son interdiction d'utilisation à des fins cosmétiques notamment en Europe depuis 2001. Extraite de la busserole ou de l'argousier, l'arbutine (β glycoside d'hydroquinone) est également reconnue pour son activité dépigmentante, mais son hydrolyse possible libérant de l'hydroquinone limite son utilisation. L'acide kojique est une autre molécule utilisée pour l'éclaircissement de la peau, mais qui peut se révéler sensibilisant et irritant pour la peau et les yeux. Par ailleurs, son instabilité au sein des formules cosmétiques rend son utilisation difficile.

Le résumé du document KR2003079111A décrit une composition blanchissante c'est-à-dire dépigmentante seulement comprenant un extrait de *Populus tremuloides. Populus tremuloides* appartient à la sous section *Trepidae* (trembles) de la section *Leuce* (peupliers blancs et trembles), constituant une des 6 sections constitutives du genre *Populus.*

Le document XP007917336 « concentrated eye cream » Database accession number 1320826 décrit une composition destinée à être appliquée dans la zone de l'oeil, la composition étant anti-ride et blanchissante. Elle contient un certain nombre de composants dont une longue liste d'extraits végétaux parmi lesquels figure un extrait d'écorce de *Populus tremuloides.* Le document ne contient aucune information concernant l'origine de l'activité blanchissante (et donc uniquement dépigmentante) du produit.

En d'autres termes, le problème que se propose de résoudre l'invention est celui de mettre au point un produit dépigmentant et/ou anti-pigmentant qui ne présente pas les inconvénients ci-dessus énoncés et qui soit en particulier, totalement dénué d'effets secondaires délétères pour la peau et/ou les poils et/ou les cheveux.

Le Demandeur a constaté que de manière tout à fait surprenante, les extraits de *Populus balsamifera* (peuplier baumier), notamment les extraits éthanolique, hydro-éthanolique ou dioxyde de carbone supercritique de bourgeons, présentaient une activité à la fois dépigmentante et anti-pigmentante meilleure que celle de l'acide kojique, et ce, quels que soient le procédé d'extraction mis en oeuvre et les solvants utilisés.

L'espèce *Populus balsamifera* appartient à la section Tacamahaca (peupliers à baume) du genre *Populus* (famille des *Salicaceae).*

Cette espèce est donc à distinguer du tremble *Populus tremuloides,* précédemment identifié, d'un point de vue taxonomique et chimiotaxonomique

*Populus balsamifera* est également parfois trouvé sous les désignations *Populus carolinensis, Populus candicans,* ou encore *Populus x gileadensis.*

On connaît également le « Balm of Gilead » (Baume de Judée, Baume de Galaad) qui peut être utilisé indistinctement pour désigner des résines (ou exsudats), des extraits issus de différentes espèces végétales (dont *P. balsamifera*), différentes parties du végétal (dont le bourgeon), différents procédés d'extraction (dont l'extraction éthanolique).

Ainsi par exemple :

- le baume de Judée originel (Old World balm of Gilead) ou baume de la Mecque (Balsam of Mecca, Balm of Mecca, Mecca balsam) est obtenu à partir de l'espèce *Commiphora gileadensis* (syn. *Commiphora opobalsamum),* famille des *Burseraceae* ;
- le baume d'Amérique (American balm of Gilead) est plutôt obtenu à partir de bourgeons d'espèces de peuplier, famille des *Salicaceae,* préférentiellement *Populus candicans,* mais aussi *P. tacamahaca, P. nigra* et *P. balsamifera* ;
- le baume du Canada (Canada Balsam, Balsam fir ou Balm of Gilead Fir) est obtenu à partir d'espèces de sapins canadiens comme *Abies balsamea* ;
- le baume obtenu à partir de certaines herbes aromatiques de la famille des *Labiateae,* comme *Dracocephalum canariense* or *Cedronella canariensis,* peut également porter le nom de « Balm of Gilead »

Cependant la composition chimique de ces différents baumes n'est pas identique, des différences qualitatives et quantitatives peuvent être observées pour les résines des différentes espèces de *Populus,* mentionnées pour le baume d'Amérique, et ces différences sont d'autant plus importantes lorsqu'il ne s'agit pas de la même famille botanique.

Le document US 20070071698 A1 décrit une composition nettoyante, protectrice vis à vis des UV contenant le « balm of Gilead » utilisé pour ses propriétés cicatrisantes. Le « balm of Gilead » désigne plus particulièrement dans ce cas le baume d'Amérique, issu des espèces *Populus candicans, Populus nigra* et *Populus balsamifera,* préférentiellement le *Populus candicans.*

Le document FR2793141 A1 décrit un mélange d'un extrait de bourgeons de *Populus nigra* et de squalane.

Le document Pearl, I.A. et al. « Studies on the leaves of the family salicaceae-XI » Phytochemistry, vol 7, n 10, 1 octobre 1968, pages 1845-1849 (XP026730369) décrit la présence d'acide cinnamique dans les feuilles de *Populus balsamifera.*

Le document KONG, Y.H. et al. « Inhibitory effects of cinnamic acid on melanin biosynthesis in skin » Biological and pharmaceutical bulletin, vol 31, n 5" 21 février 2008, pages 946-948, (XP002624485) décrit l'effet inhibiteur de la tyrosinase par l'acide cinnamique. L'IC50 s'avère être supérieure à celle de l'acide kojique ce qui signifie que l'effet dépigmentant de l'acide cinnamique pur est inférieur à celui de l'acide kojique.

A la connaissance du Demandeur, l'activité dépigmentante des extraits de *Populus balsamifera* n'a pas été décrite et on ne peut présager une telle activité au motif qu'il est connu que le peuplier contient de l'acide cinnamique. Cette molécule, appartenant à la classe des acides phénylpropanoïques, est biosynthétisée par une grande partie des végétaux supérieurs et joue le rôle d'intermédiaire dans la biosynthèse de tous les composés phénylpropanoïques notamment. Dans l'extrait de *Populus,* elle fait partie d'un totum de molécules et c'est l'ensemble de ces molécules associées qui caractérise les propriétés de l'extrait. L'acide cinnamique peut avoir une activité nulle ou contributive pour le totum, mais ne porte en aucun cas seul les propriétés de l'extrait dans le cadre de l'invention qui met en évidence une IC50 de l'extrait (totum) inférieure à celle de l'acide kojique.

En d'autres termes, l'invention a pour objet l'utilisation non thérapeutique d'un extrait de bourgeons de *Populus balsamifera* obtenu par extraction en milieu éthanolique, hydro-éthanolique ou dioxyde de carbone supercritique comme agent dépigmentant et/ou anti-pigmentant de la peau et/ou des cheveux et/ou des poils dans une composition cosmétique et/ou dermatologique.

En pratique, l'extrait est obtenu par une première étape d'extraction solide/liquide, suivie d'une seconde étape de séparation solide/liquide, puis d'une troisième étape de récupération de la phase liquide.

L'extraction solide/liquide peut être effectuée par différentes techniques bien connues de l'homme du métier, telles que macération, re-macération, digestion, macération dynamique, décoction, extraction en lit fluide, extraction assistée par micro-ondes, extraction assistée par ultra-sons, extraction à contre courant, percolation, re-percolation, lixiviation, extraction sous pression réduite, diacolation, extraction par fluide supercritique, extraction par eau subcritique, extraction solide-liquide sous reflux continu (soxhlet).

Selon une autre caractéristique, l'extraction solide/liquide est effectuée à partir avantageusement de bourgeons sous forme fraîche ou sèche, les bourgeons pouvant se présenter en outre sous forme entière, concassée, broyée, ou cryobroyée.

Par ailleurs, le solvant d'extraction correspondant à la phase liquide est un solvant aqueux ou organique. De manière générale, tous les solvants peuvent être utilisés quelle que soit leur polarité. La gamme de polarité des solvants utilisables est définie par des valeurs de constante diélectrique, ou permittivité relative (notée $\varepsilon'$ ou $\varepsilon_R$), déterminées à 20°C, comprises entre 1 et 81. Le solvant est d'autant plus polaire que la valeur de la constante diélectrique est élevée. A l'inverse une valeur faible de la constante diélectrique caractérise un solvant à caractère apolaire. Le solvant d'extraction peut donc être choisi dans le groupe comprenant l'eau (eau douce $\varepsilon_R=78.5$, eau distillée $\varepsilon_R=81$), les alcools tels que éthanol ($\varepsilon_R=24$), méthanol, etc, les glycols tels que le propylèneglycol ($\varepsilon_R=32$), le propanediol, le butylèneglycol, etc, la glycérine ($\varepsilon_R=42$), seuls ou en mélange. Il peut être également choisi dans une gamme moins polaire comme des alcools en $C_3$ ou plus, des cétones (acétone, butanone, etc), des esters (acétate d'éthyle $\varepsilon_R=6$, etc), et également dans la gamme de solvants apolaires comme l'heptane ($\varepsilon_R=1.9$), le $CO_2$ supercritique ($\varepsilon_R=1$), Bien entendu, les solvants organiques non autorisés en cosmétique dans une forme liquide de l'extrait sont ensuite éliminés de l'extrait final par des techniques adaptées citées par la suite de manière non limitative.

En pratique le ratio plante/solvant appliqué pour l'étape d'extraction est compris entre 1/99 et 80/20. L'extraction s'opère à une température comprise entre 2 et 100°C, préférentiellement entre 20 et 80°C, pendant quelques minutes à plusieurs jours en fonction de la méthode d'extraction utilisée.

De manière à optimiser l'extraction des composés actifs tout en protégeant ces composés de l'oxydation par l'oxygène de l'air, l'étape d'extraction solide/liquide peut être réalisée sous agitation et sous atmosphère d'azote.

Selon l'invention, l'extraction solide/liquide est suivie d'une étape de séparation solide/liquide, l'objectif étant de récupérer la phase liquide contenant la matière active. Cette séparation peut être effectuée par toute technique connue de l'homme du métier, en particulier l'égouttage, le pressage, l'essorage, la centrifugation ou la filtration.

Selon un autre mode de réalisation, l'étape de séparation liquide/solide est suivie d'une étape de concentration, laquelle permet d'obtenir une forme liquide concentrée ou semi-solide selon le facteur de concentration. En pratique, l'étape de concentration est effectuée par évaporation sous pression réduite ou osmose inverse.

Postérieurement à l'étape de séparation solide/liquide et dans un autre mode de réalisation, l'extrait obtenu est fractionné, enrichi ou purifié par différentes techniques telles que la filtration membranaire, l'extraction liquide/liquide ou la chromatographie préparative.

Postérieurement aux étapes de concentration ou de fractionnement, l'extrait peut être solubilisé dans un solvant autorisé en cosmétique, tels que par exemple l'eau, les glycols, la glycérine, l'éthanol, les triglycérides à chaînes moyennes, des esters d'acides gras et des huiles végétales.

Enfin, en vue d'un conditionnement stérile ou non stérile, les étapes de clarification et/ou de concentration peuvent être suivies d'une ou plusieurs étapes de filtration jusqu'à une filtration finale stérilisante à $0.22\mu$m dans le cas d'extrait contenant de l'eau. Préalablement à l'étape de filtration finale, des additifs tels que conservateurs et antioxydants connus de l'homme du métier peuvent être incorporés dans l'extrait liquide pour garantir sa stabilité.

Pour obtenir un extrait sous forme sèche, l'extrait obtenu à l'issue d'une des étapes précédemment énoncées, est séché, avec ou sans agent texturant (tel que l'amidon, les maltodextrines, les sirops de glucose...), par lyophilisation, atomisation ou évaporation sous pression réduite.

L'extrait de l'invention peut se présenter sous forme solide, semi solide ou liquide.

Ainsi, l'extrait a une teneur en matière sèche comprise entre 1 et 950 g/kg, selon le procédé de fabrication utilisé.

Dans un mode de réalisation avantageux, l'extrait se présente sous forme liquide et présente une teneur en matière sèche comprise entre 1 et 100g/kg, avantageusement entre 10 et 50 g/kg. L'extrait est dissous dans un solvant cosmétique choisi avantageusement dans les groupes des glycols, préférentiellement le 1,3-propanediol (Nom INCI: Propanediol), ou un mélange de triglycérides à chaîne moyenne, préférentiellement le mélange portant le nom INCI Caprylic/Capric Triglycéride (tels que Labrafac® CC, Mygliol® 810 ou 812, ...).

L'invention a également pour objet un extrait de *Populus balsamifera* pour une utilisation comme agent dépigmentant et/ou anti-pigmentant.

L'extrait est donc destiné à être utilisé dans le domaine cosmétique et/ou dermatologique par voie topique, comme agent dépigmentant et/ou anti-pigmentant.

En pratique, l'extrait sous forme liquide représente entre 0.1 % et 10 % en poids de la composition cosmétique et/ou dermatologique, préférentiellement entre 0.5 % et 5 %.

En pratique, l'extrait sous forme semi-solide ou solide représente entre 0.01% et 5% en poids de la composition cosmétique et/ou dermatologique, préférentiellement entre 0.05% et 2%.

La composition cosmétique et/ou dermatologique selon l'invention peut se présenter sous toutes les formes galéniques normalement utilisées pour une application topique sur la peau ou les cheveux ou les poils, notamment sous forme presque anhydre, d'une émulsion huile-dans-eau ou eau-dans-huile ou multiple, d'une émulsion siliconée, d'une microémulsion, d'une nanoémulsion, d'un gel ou d'une solution nettoyante de type shampooing.

Cette composition peut être plus ou moins fluide et avoir l'aspect entre autres d'une crème blanche ou colorée, d'une pommade, d'un lait, d'une lotion, d'un sérum, d'un gel, de liposomes, d'un masque, de patchs transdermiques ou de lingettes imbibées.

La composition cosmétique et/ou dermatologique peut contenir les adjuvants habituels dans les domaines cosmétique et dermatologique, tels que les matières grasses, les tensioactifs détergents et/ou conditionnants, les émulsionnants et co-émulsionnants, les gélifiants hydrophiles ou lipophiles, les actifs hydrophiles ou lipophiles, les conservateurs, les antioxydants, les solvants, les agents exfoliants, les parfums, les charges, les filtres hydrophiles et lipophiles, les matières colorantes, les neutralisants, les agents pro-pénétrants, et les polymères.

Les quantités de ces différents adjuvants sont celles classiquement utilisées dans les domaines considérés, et par exemple de 0.01 à 30% du poids total de la composition. Ces adjuvants, selon leur nature, peuvent être introduits dans la phase grasse ou dans la phase aqueuse.

Comme matières grasses utilisables dans l'invention, on peut utiliser les huiles minérales, les huiles d'origine animale (lanoline), les huiles végétales, les huiles de synthèse (isopropyl myristate, octyldodecyl, isostearyl isostearate, decyl oleate, isopropyl palmitate), les huiles siliconées (cyclomethicone, dimethicone) et les huiles fluorées. On peut utiliser comme matières grasses des alcools gras, des acides gras, des cires et des gommes et en particulier les élastomères de silicone.

Comme tensioactifs détergents et/ou conditionnants utilisables dans l'invention, on peut citer les tensioactifs non ioniques, anioniques, cationiques ou amphotères, et leurs mélanges, rencontrés classiquement dans les formules de type shampooing. Par exemple les alkylsulfates, les alkyléthersulfates tel que le lauryl éther sulfate de sodium, les alkyl bétaïnes telle que la cocamidopropylbetaïne, ou les sels d'ammonium quaternaires.

Comme émulsionnants et co-émulsionnants utilisables dans l'invention, on peut citer par exemple les esters de polyglycérols et d'acide gras, les esters de sucrose et d'acide gras, les esters de sorbitane et d'acide gras, les esters d'acide gras et de sorbitane oxyéthylénés, les ethers d'alcool gras et de PEG, les esters de glycérol et d'acide gras, les alkyl sulfates, les alkyl ether sulfates, les alkyl phosphates, les alkyl polyglucosides, les dimethicone copolyols.

Comme gélifiants hydrophiles, on peut citer en particulier les polymères carboxyvinyliques (carbomers), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, les amidons et leurs dérivés, les argiles et les copolymères d'acide 2-acrylamido-2-méthylpropane.

Comme gélifiants lipophiles, on peut citer les argiles modifiées comme les bentones, les sels métalliques d'acides gras, la silice hydrophobe et l'éthylcellulose.

La composition cosmétique et/ou dermatologique peut également contenir des actifs. Comme actifs, on peut utiliser notamment les anti-radicalaires ou plus généralement les antioxydants, les émollients, les hydratants, les anti-séborrhéiques, les anti-inflammatoires, les anti-acnéiques, les agents kératolytiques et/ou desquamants, les agents anti-rides et tenseurs, les agents drainants, les agents anti-irritants, les agents apaisants, les vitamines et leurs mélanges, les agents matifiants, les actifs anti-âge tel que le rétinol, les cicatrisants, les antiseptiques et les huiles essentielles.

Comme conservateurs utilisables selon l'invention, on peut citer les acides benzoïque, sorbique, propionique, salicylique, dehydroacétique et leurs sels; l'alcool benzylique, l'éthylhexylglycérine, les parabens, leurs sels et esters ;

le triclosan ; l'imidazolidinyl urée ; le phenoxyethanol ; la DMDM hydantoïne ; le diazolidinyl urée ; la chlorphenesine.

Comme antioxydants utilisables selon l'invention, on peut citer les agents chelatants tels que l'EDTA et ses sels, le metabisulfite de sodium, les acides salicylique, ascorbique et citrique et leurs sels, le tartrate de sodium, le gluconate de sodium, les caroténoïdes et les tocophérols.

Comme solvants utilisables selon l'invention, on peut citer l'eau, l'éthanol, la glycérine, le propylène glycol, le propanediol, le butylène glycol, le sorbitol.

Comme agents exfoliants utilisables selon l'invention, on peut citer les exfoliants chimiques tels que les AHA, et les exfoliants physiques tels que les poudres naturelles ou synthétiques.

Comme charges utilisables selon l'invention, on peut citer le talc, le kaolin, le mica, la serecite, le magnesium carbonate, l'aluminium silicate, le magnesium silicate, les poudres organiques telles que le nylon.

Comme filtres utilisables selon l'invention, on peut citer les filtres UVA et UVB classiquement utilisés tels que la benzophenone-3, le butyl methoxydibenzoyl methane, l'octocrylène, l'octyl methoxycinnamate, le 4-methylbenzylidene camphor, l'octyl salycylate, le tacephthalydene dicamphor sulfanic acid, le drométrizole trisiloxane, le methylene bis-benzotriazolyl tetramethylbutylphenol, le bis-ethylhexyloxyphenol methoxyphenyl triazine, l'ethylhexyl triazone, et le Diethylamino Hydroxybenzoyl Hexyl Benzoate. On citera également les filtres physiques $TiO_2$ et ZnO sous leurs formes micrométriques et nanométriques, enrobés ou non enrobés.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Comme neutralisants utilisables selon l'invention, on peut citer la soude, la triethanolamine, l'aminomethyl propanol, l'hydroxyde de potassium.

Comme agents pro pénétrants utilisables selon l'invention, on peut citer les alcools et glycols (éthanol, propylène glycol), l'éthoxydiglycol, les alcools et acides gras (acide oléique), les esters d'acides gras, le dimethyl isosorbide.

L'invention a également pour objet un procédé de traitement cosmétique et/ou dermatologique de dépigmentation et/ou anti-pigmentation de la peau et/ou des cheveux et/ou des poils consistant à appliquer localement une quantité efficace de la composition précédemment décrite, par voie topique.

L'invention et les avantages qui en découlent ressortiront bien des exemples de réalisation suivants :

Exemple 1 : Fabrication d'un extrait de *Populus balsamifera*

- Exemple 1 a : sous la forme d'un lyophilisat

On mélange 1 partie de bourgeons secs de *Populus balsamifera* et 9 parties d'un mélange éthanol/eau 50:50v/v. Le mélange est extrait au reflux pendant 3h puis on filtre l'extrait. Les tourteaux de bourgeons sont lavés à température ambiante pendant 2h par le même mélange de solvants, puis on filtre l'extrait. Après réunion des 2 extraits, on concentre ce mélange sous pression réduite dans un évaporateur afin d'éliminer l'éthanol. On laisse décanter le concentrat aqueux à température ambiante pendant 2h. Le surnageant est prélevé, filtré puis lyophilisé. On obtient ainsi une poudre de couleur marron caractérisée par une teneur en matière sèche d'environ 950 g/kg.

- Exemple 1 b :Au moyen d'une résine hydro-éthanolique

On mélange 1 partie de bourgeons secs et 9 parties du mélange éthanol/eau 50:50v/v. Le mélange est extrait au reflux pendant 3h puis on filtre l'extrait. Les tourteaux de bourgeons sont lavés à température ambiante pendant 2h par le même mélange de solvants, puis on filtre l'extrait. Après réunion des 2 extraits, on concentre ce mélange sous pression réduite dans un évaporateur afin d'éliminer l'éthanol. On laisse décanter le concentrat aqueux à température ambiante pendant 2h. On soutire la fraction semi-solide (le surnageant aqueux peut être traité comme dans l'exemple 1). On obtient ainsi un extrait semi-solide de couleur brun ambré caractérisé par une teneur en matière sèche d'environ 800 g/kg.

- Exemple 1 c :Au moyen d'une résine éthanolique

On mélange 1 partie de bourgeons secs et 9 parties d'éthanol. Le mélange est extrait au reflux pendant 3h puis on filtre l'extrait. Les tourteaux de bourgeons sont lavés à température ambiante pendant 2h par le même mélange de solvants, puis on filtre l'extrait. Après réunion des 2 extraits, on concentre ce mélange sous pression réduite dans un évaporateur afin d'éliminer l'éthanol. On obtient ainsi un extrait mou de couleur brun rouge caractérisé par une teneur en matière sèche d'environ 800 g/kg.

- Exemple 1 d :par extraction par $CO_2$ supercritique

On mélange les bourgeons secs broyés dans l'extracteur. Ces bourgeons sont extraits par diffusion du dioxyde de carbone porté à l'état supercritique, en appliquant une température et une pression supérieures aux conditions du point critique, c'est à dire T > 31°C, P > 73 bars. En fin d'extraction le fluide supercritique chargé des matières végétales extraites est détendu à l'état gazeux, et on collecte dans le séparateur les matières extraites rendues insolubles dans le dioxyde de carbone gazeux. On obtient ainsi un extrait semi-solide translucide de couleur jaune orangé caractérisé par une teneur en matière sèche d'environ 900 g/kg.

Exemple 2 : Mise en évidence de l'activité dépigmentante

*Méthodologie*

Le modèle biologique utilisé est une culture de mélanocytes issus d'une lignée de mélanome murin B16, très réactif en terme de modulation de la pigmentation. Les cellules sont cultivées en plaque 96 puits en milieu de culture complémenté ou pas par la NDP-MSH, dérivé stable de l'$\alpha$-MSH, hormone naturelle de stimulation de la mélanogenèse.
La présence de NDP-MSH caractérise la pigmentation UV induite et permet la mesure de l'effet anti-pigmentant. L'absence de NDP-MSH dans le test, caractérise la pigmentation constitutive, et permet la mesure de l'effet dépigmentant. A titre d'exemple, les mélanocytes sont incubés pendant 72 h avec les extraits de bourgeons de *Populus balsamifera* ou l'acide kojique, référence positive choisie pour ce test.
En fin d'incubation, la mélanine totale (intracellulaire et extracellulaire) est quantifiée par mesure de l'absorbance à 405nm dans chaque puits de culture contre une gamme étalon de mélanine.

*Expression des résultats*

- Le bruit de fond (contrôle d'interférence lié à la couleur des produits testés), mesuré dans les puits sans cellule, a été retranché aux valeurs brutes mesurées.
- Pour chaque condition (stimulée ou non), on calcule les % de mélanine par rapport au témoin, et l'IC50 est estimée pour chaque produit étudié (IC50 : concentration (exprimée en $\mu$g/ml) pour laquelle 50% de la synthèse de mélanine est inhibée).
- Calcul des % d'inhibition de la synthèse de mélanine selon les formules suivantes :

- Conditions non stimulées

$$\text{Inhibition (\%)} = 100 - \left[ \frac{\text{Valeur}}{\text{Moyenne du témoin}} \times 100 \right]$$

- Conditions stimulées

$$\text{Inhibition (\%)} = \frac{\text{Moyenne Témoin stimulé - Valeur}}{\text{Moyenne Témoin stimulé - Moyenne Témoin basal}} \times 100$$

*Mesure de viabilité*

En fin d'incubation, la viabilité cellulaire est mesurée dans chacun des puits de culture par un test MTT, test colorimétrique basé sur la transformation du MTT (3(4,5-diméthylthiazol-2-yl)-2,5 diphényl tétrazolium bromide) par la succinyldehydrogénase mitochondriale en cristaux bleu formazan. Le taux de transformation du MTT est le reflet de l'activité métabolique de la cellule, et est corrélé au nombre de cellules vivantes.
Expression des résultats : % de viabilité par rapport au témoin selon la formule :

$$\text{Viabilité (\%)} = (\text{DO}_{\text{composé}} / \text{DO}_{\text{témoin}}) \times 100$$

Ce contrôle *a posteriori* de la viabilité cellulaire est réalisé afin de vérifier que les activités dépigmentantes mesurées pour les produits ne sont pas liées à une cytotoxicité vis à vis des mélanocytes.

*Résultats*

| | | IC 50 (µg/ml) | |
|---|---|---|---|
| **Produit testé** | | *Sans NDPMSH* | *Avec NDPMSH* |
| Acide kojique | | 650 | 70 |
| Extrait *P. balsamifera "Lyophilisat"* (exemple 1a) | | 12,8 | 12 |
| Extrait *P. balsamifera "Résine hydro-éthanolique"* (exemple 1b) | | 14 | 14,4 |
| Extrait *P. balsamifera "Résine éthanolique"* (exemple 1c) | | 15,3 | 11,8 |
| Extrait *P. balsamifera "Extrait CO_2S"* (exemple 1d) | | 10,6 | 22,1 |

Les extraits de bourgeons de *Populus balsamifera* démontrent bien leur efficacité pour inhiber la production de mélanine, tant dans les conditions de stimulation de la mélanogenèse (avec NDPMSH, c'est-à-dire pigmentation UV induite) qu'en conditions basales (sans NDPMSH, pigmentation constitutive).

Quelle que soit la méthode d'extraction, ils présentent tous une efficacité supérieure à celle de l'acide kojique. En effet, l'IC 50 des extraits est toujours inférieure à celle mesurée pour l'acide kojique, que ce soit en condition de mélanogenèse stimulée comme dans les conditions basales.

Exemple 3 : Compositions cosmétiques

Crème blanchissante pour la peau

| Ingrédients | Quantité (%) |
|---|---|
| Cetyl Alcohol, Glyceryl Stearate, PEG-75 Stearate, Ceteth-20, Steareth-20 | 8.00 |
| Octyl Methoxycinnamate | 10.00 |
| Cetyl Alcohol | 1.50 |
| Bis-Ethylhexyloxyphenol Methoxyphenyl Triazine | 1.50 |
| Phenoxyethanol, Methylparaben, Butylparaben, Ethylparaben, Propylparaben | 0.70 |
| Gomme xanthane | 0.40 |
| Ascorbic Acid | 0.20 |
| Salicylic Acid | 2.00 |
| Lactic Acid (85%) | 1.00 |
| Ethoxydiglycol | 1.50 |
| **Extrait de l'exemple 1b** | **1.00** |
| Hydroxide de sodium (10%) | 4.50 |
| Methyl Bis-Benzotriazolyl Tetramethylbutylphenol | 5.00 |
| Eau | Qsp 100 |

Crème décolorante pour les cheveux

| Ingrédients | Quantité (%) |
|---|---|
| Cetyl Alcohol, Glyceryl Stearate, Ceteth-20, Steareth-20 | 6.00 |
| Behenyl Alcohol | 2.00 |
| Cetearyl Alcohol | 2.00 |
| Octyldodecyl Myristate | 4.00 |
| Hydrogenated Polydecene | 4.00 |
| Phenoxyethanol, Methylparaben, Butylparaben, Ethylparaben, Propylparaben | 0.50 |
| Phosphoric Acid (10%) | 0.20 |
| Acetanilide (1.5%) | 0.50 |
| Hydrogen peroxide 110 vol | 15.00 |
| **Extrait de l'exemple 1c** | **1.00** |
| Eau | Qsp 100 |

Shampooing décolorant

| Ingrédients | Quantité (%) |
|---|---|
| Sodium Laureth Sulfate (40%) | 35.00 |
| Polyquaternium-7 | 1.00 |
| Cocamide DEA | 3.50 |
| Methylchoroisothiazolinone, Methylisothiazolinone | 0.10 |
| Cocamidopropyl Betaine | 3.00 |
| Disodium EDTA | 0.10 |
| Acrylate/C10-30 Alkyl Acrylates Crosspolymer | 0.40 |
| Hydroxyde de Sodium (10%) | 0.20 |
| **Extrait de l'exemple 1b** | **2.00** |
| Eau | Qsp 100 |

**Revendications**

1. Utilisation non thérapeutique d'un extrait de bourgeons de *Populus balsamifera* obtenu par extraction en milieu éthanolique, hydro-éthanolique ou dioxyde de carbone supercritique comme agent dépigmentant et/ou anti-pigmentant de la peau et/ou des cheveux et/ou des poils dans une composition cosmétique et/ou dermatologique.

2. Utilisation selon la revendication 1, **caractérisée en ce que** l'extrait est obtenu par extraction au moyen d'un solvant présentant une permittivité relative $\varepsilon_R$ comprise entre 1 et 81.

3. Utilisation selon la revendication 1, **caractérisée en ce que** l'extrait se présente sous forme liquide, semi-solide ou solide.

4. Utilisation selon la revendication 1, **caractérisée en ce que** l'extrait se présente sous forme liquide et présente une teneur en matière sèche comprise entre 1 et 100g/kg, avantageusement entre 10 et 50 g/kg.

5. Utilisation selon la revendication 1, **caractérisée en ce que** l'extrait est dissous dans un solvant choisi dans les groupes des glycols, avantageusement le propanediol, des triglycérides à chaîne moyenne, avantageusement le mélange caprylic/capric triglyceride.

6. Utilisation selon la revendication 1, **caractérisée en ce que** :

- l'extrait sous forme liquide représente entre 0.1 % et 10 % en poids de la composition, préférentiellement de 0.5 à 5%,
- l'extrait sous forme semi-solide ou solide représente entre 0.01% et 5% en poids de la composition, avantageusement entre 0.05% et 2%.

7. Procédé de traitement cosmétique et/ou dermatologique de dépigmentation et/ou anti-pigmentation de la peau et/ou des cheveux et/ou des poils consistant à appliquer par voie topique la composition mentionnée dans l'une des revendications 1 à 6.

**Patentansprüche**

1. Nichttherapeutische Verwendung eines Extrakts aus Knospen von *Populus balsamifera,* der durch Extraktion in superkritischem Ethanol-, Wasser/Ethanol- oder Kohlendioxidmedium gewonnen ist, als Depigmentierungsmittel und/oder Antipigmentierungsmittel für die Haut und/oder Kopfhaare und/oder Körperhaare in einer kosmetischen und/oder dermatologischen Zusammensetzung.

2. Verwendung nach Anspruch 1, **dadurch gekennzeichnet, dass** der Extrakt durch Extraktion mittels eines Lösungsmittels gewonnen ist, das eine relative Permittivität $\varepsilon_R$ aufweist, die zwischen 1 und 81 beträgt.

3. Verwendung nach Anspruch 1, **dadurch gekennzeichnet, dass** sich der Extrakt in flüssiger, halbfester oder fester Form darstellt.

4. Verwendung nach Anspruch 1, **dadurch gekennzeichnet, dass** sich der Extrakt in flüssiger Form darstellt und einen Gehalt an Trockenstoff aufweist, der zwischen 1 und 100 g/kg, vorteilhafter Weise zwischen 10 und 50 g/kg beträgt.

5. Verwendung nach Anspruch 1, **dadurch gekennzeichnet, dass** der Extrakt in einem Lösungsmittel aufgelöst ist, das aus den Gruppen der Glykole, vorteilhafter Weise Propandiol, mittelkettiger Triglyceride, vorteilhafter Weise dem Gemisch Capryl/Caprintriglycerid ausgewählt ist.

6. Verwendung nach Anspruch 1, **dadurch gekennzeichnet, dass**:

- der Extrakt in flüssiger Form zwischen 0,1 Gew.-% und 10 Gew.-% der Zusammensetzung, bevorzugt von 0,5 bis 5% darstellt,
- der Extrakt in halbfester oder fester Form zwischen 0,01 Gew.-% und 5 Gew.-% der Zusammensetzung, vorteilhafter Weise zwischen 0,05% und 2% darstellt.

7. Verfahren zur kosmetischen und/oder dermatologischen Behandlung zur Depigmentierung und/oder Antipigmentierung der Haut und/oder der Kopfhaare und/oder der Körperhaare, das darin besteht, die in einem der Ansprüche 1 bis 6 erwähnte Zusammensetzung auf topischem Wege aufzutragen.

**Claims**

1. Non-therapeutic use of an extract of buds of *Populus balsamifera*, obtained via extraction in an ethanolic, hydroethanolic or supercritical carbon dioxide environment, as a depigmenting and/or anti-pigmenting agent for skin and/or head hair and/or body hair in a cosmetic and/or dermatological composition.

2. Use according to Claim 1, wherein the extract is obtained via extraction using a solvent that has a relative permittivity $\varepsilon_R$ between 1 and 81.

3. Use according to Claim 1, wherein the extract is in the form of a liquid, semi-solid or solid.

4. Use according to Claim 1, wherein the extract is in the form of a liquid and has a dry material content of between 1 and 100 g/kg, preferably between 10 and 50 g/kg.

5. Use according to Claim 1, wherein the extract is dissolved in a solvent selected from the groups of glycols, preferably propanediol, or medium-chain triglycerides, preferably the caprylic/capric triglyceride compound.

6. Use according to Claim 1, wherein:

   - The extract in the form of a liquid represents between 0.1% and 10% of the weight of the composition, preferably 0.5-5%,
   - The extract in its semi-solid or solid form represents between 0.01% and 5% of the weight of the composition, preferably between 0.05% and 2%.

7. Cosmetic and/or dermatological treatment for the depigmentation and/or anti-pigmentation of the skin and/or head hair and/or hair, consisting of the topical application of the composition mentioned in any of the claims 1 to 6.

## RÉFÉRENCES CITÉES DANS LA DESCRIPTION

**Documents brevets cités dans la description**

- KR 2003079111 A
- US 20070071698 A1
- FR 2793141 A1

**Littérature non-brevet citée dans la description**

- **PEARL, I.A. et al.** Studies on the leaves of the family salicaceae-XI. *Phytochemistry,* 01 Octobre 1968, vol. 7 (10), 1845-1849
- **KONG, Y.H. et al.** Inhibitory effects of cinnamic acid on melanin biosynthesis in skin. *Biological and pharmaceutical bulletin,* 21 Février 2008, vol. 31 (5), 946-948